# NEW EUROPEAN PATENT SPECIFICATION

(11) **EP 2 520 301 B2**
(45) Date of publication and mention of the opposition decision: **03.03.2021**
(45) Mention of the grant of the patent: 10.05.2017
(21) Application number: 10837927.2
(22) Date of filing: 14.12.2010
(51) Int. Cl.: A61K 9/14, A61K 31/565, A61K 31/57, A61K 9/10, A61P 5/34, A61K 45/06, A61P 5/30

(54) **PARENTERAL PHARMACEUTICAL FORMULATION IN SUSPENSION, HAVING SUSTAINED RELEASE, IN LOW AND ULTRALOW DOSAGE, IN HORMONAL THERAPY IN THE CLIMACTERIC SYNDROME**
PARENTERAL VERABREICHTE PHARMAZEUTISCHE FORMULIERUNG MIT VERZÖGERTER FREISETZUNG IN EINER SUSPENSION IN GERINGER ODER ULTRAGERINGER DOSIERUNG ZUR VERWENDUNG FÜR HORMONTHERAPIEN IM KLIMAKTERIUM
FORMULATION PHARMACEUTIQUE PARENTÉRALE EN SUSPENSION, À LIBÉRATION SOUTENUE, À DOSAGE FAIBLE ET ULTRA-FAIBLE, UTILISÉE DANS L'HORMONOTHÉRAPIE DU SYNDROME MÉNOPAUSIQUE

(30) Priority: 15.12.2009 MX 2009013768
(43) Date of publication of application: 07.11.2012
(73) Proprietor: Techsphere, S.A. De Cv., CP. 03100 México D.F. (MX)
(72) Inventor: ÁNGELES URIBE, Juan, C.P. 03100 México D.F. (MX); SAVOIR VILBOEUF, John Claude, C.P. 03100 México D.F. (MX)
(74) Representative: Forresters IP LLP
(86) International application number: PCT/MX2010/000154
(87) International publication number: WO 2011/074931

(56) References cited:
- EP-A2- 1 820 494
- WO-A2-2004/110408
- US-A- 5 360 616
- US-A- 5 643 604
- US-A- 6 077 531
- US-B1- 6 287 693
- CORTES-BONILLA et al.: "Treatment of menopausal symptoms with three low-dose continuous sequential 17(3-estradiol/progesterone parenteral monthly formulations using novel non- polymeric microsphere technology", Gynecol Endocrinol, vol. 31, no. 7, 3 July 2015 (2015-07-03), pages 552-559,
- MITTAL et al.: "Estradiol loaded PLGA nanoparticles for oral administration: effect of polymer molecular weight and copolymer composition on release behavior in vitro and in vivo", Control Release, vol. 119, no. 1, 14 May 2007 (2007-05-14), pages 77-85,
- GARZA-FLORES et al.: "A pilot study on the assessment of a progesterone/estradiol sustained release as once-a-month-injectable contraceptive", Contraception, vol. 44, no. 1, July 1991 (1991-07), pages 45-59,
- NALAWADE T. M. et al.: "Bactericidal activity of propylene glycol, glycerine, polyethylene glycol 400, and polyethylene glycol 1000 against selected", J Int Soc Prev Community Dent., vol. 5, no. 2, April 2015 (2015-04), pages 114-119,
- "Ultrasonic" In: Hao Feng et al: "Ultrasound Technologies for food and bioprocessing", 2011, Springer ISBN: 978-1-4419-7471-6 page 138,
- World Health Organization: "Quality Assurance of pharmaceuticals", 2007, WHO page 222,
- Jameela et al: JOURNAL OF CONTROLLED RELEASE, vol. 52, no. 1-2, March 1998 (1998-03), pages 17-24,
- Ettinger et al: Obstetrics & Gynecology, vol. 104, no. 3, September 2004 (2004-09),
- Johnson et al: Obstetrics & Gynecology, vol. 105, no. 4, April 2005 (2005-04),

## Description

### FIELD OF THE INVENTION

A parenteral pharmaceutical formulation in suspension, of sustained release, in low and ultra-low dose, for therapeutic use of hormonal therapy during climacteric syndrome.

### Prior Art

Climacteric syndrome or menopause is a physiological event that marks the end of reproductive life in women, it occurs universally to all women that reach their middle age. Globally (men and women), this represented in 1990, 10% of the planet's population (5-8% in underdeveloped countries and near to 15% in industrialized countries. Hill, K. 1996. The demography of menopause. Maturitas. Vol. 23, No. 2: 113-127). Between 1990 and 2000, some 24.5 million women went through menopause each year, in China only; they were near to 5 million per year. In the year 2020 this amount will be doubled, with half of those women in Asia. The median of the age at which menopause is reached in Europe and the United States is 50 years (Ginsberg, J. 1991. What determines the age at the menopause? Br. Med. J. Vol. 302, No. 6788:1288-1289), while in India and the Philippines it is reached at the median age of 44 years (Goodman, M.J., et al. 1985. Menarche, pregnancy, birth spacing and menopause among the Agta women foragers of Cagayan province, Luzon, the Philippines. Ann. Hum. Biol. Vol. 12, No. 2: 169-177; Sharma, V.K., & Saxena, M. S. 1987. Climacteric symptoms: A study in the Indian context. Maturitas. Vol. 3, No. 1:11-20), in the United Arab Emirates is 48 years (Rizk, D.E., et al. 1998. The age and symptomatology of natural menopause among United Arab Emirates women. Maturitas Vol. 29, No. 3: 197-202), the same as Turkey, Israel, and most part of Asia, Mexico, Nigeria and Ghana (Khwak, K.T. 1992, Epidemiology of the menopause, Br. Med. Bull. Vol. 48: 249-261). Menopause is defined as the permanent cessation of menstruation due to the loss of follicular ovarian function, there are many symptoms associated to it, of variable frequency and severity, among them, vasomotor symptoms, hot flashes, night perspiration, vaginal dryness, bad humor, irritability, anxiety, depression, insomnia, headaches, urinary disorders, pain in articulations and fatigue (Ringa, V. 2000. Menopause and treatments. Quality of life research. Vol. 9: 695-707). Menopause corresponds to the last menstrual period that occurs due to the loss of ovarian activity and is identified when 12 months of amenorrhea have elapsed (Malacara, J.M. 2003. Menopausia: Nuevas evidencias, nuevos enigmas. Rev. Endocrinol y Nutr. Abr.-Jun. Vol. 11, No. 2: 61-72). It only occurs in the human species, although in some mammalians such as lions or baboons, ovarian function decreases (Packer, C., et al. 1998. Reproductive cessation in female animals. Nature. Vol. 392: 807-811).

In the treatment of the symptomatology and discomfort associated with climacteric, hormonal replacement has been used, nevertheless, such treatments have been correlated with the appearing of some forms of cancer (Zárate A., & Hernández Valencia, M. 2002. Terapia de reemplazo hormonal en mujeres menopáusicas tratadas por cancer mamario. Rev Med. IMSS Vol. 40, No. 5: 369-371; Cóppola, Francisco, et al. 2004. La terapia hormonal en la post-menopausia y las promesas incumplidas. Rev. Méd. Uruguay. Vol. 20, No. 2: 130-135; Schairer, C., et al. 2000, Menopausal estrogen ad estrogen-progestin replacement therapy and breast cancer risk. JAMA. Vol. 283, No. 4: 485-491), while other therapies have demonstrated to be much less effective. The aim of an effective compound or formulation for the intended purposes is to achieve estradiol balance (a molecule that has an effective action in the uterus, making it to continue with its function, however, it causes an increase in the risk of suffering uterine cancer), with a uterus protector such as progesterone (nevertheless, it seems to increase the possibility of suffering from breast cancer) on this account, we thought of using low and ultra-low doses in replacement therapy.

Low and ultra-low dose estrogen. In the current medical literature, this expression covers any product made from estrogen, natural or synthetic, containing half or less the amount that is usually used for replacement hormone therapy either oral or transdermal during climacteric or post-menopause, while ultra-low dose is equivalent to the fourth part of the standard dose, that is, 1.0mg of estradiol and 20.0mg of progesterone for a low dose and, between 0.25 to 0.50 mg of estradiol and 15.0 mg of progesterone for an ultra-low dose (Velasco-Murillo V. 2006. Estrógenos a dosis bajas y estrógenos de síntesis. ¿Opciones para el remplazo hormonal en el climaterio? Rev Med Inst Mex Seguro Soc 2007; Vol. 45, No. 4: 381-387. Carranza Lira, S. 2008. Dosis baja de terapia hormonal durante el climaterio. Ginecol. Obstet. Mex. Vol. 76, No. 5: 267-274). Hormonal doses of 1mg of estradiol and 20mg of progesterone, or 0.5mg of estradiol and 15 mg of progesterone for intramuscular monthly administration, proposed in this invention, comply with the criteria for low doses for use in hormonal replacement therapy during climacteric, for both types of hormones. The benefits from the treatment with estrogen at low doses are appreciated with the results in table 1 below:

**Table 1. Effectiveness and side effects of estrogen at standard and low doses.**

| | Standard Dose | Low Dose |
|---|---|---|
| Effectiveness | | |
| Suppression of vasomotor disorders | 80 to 90% | 60 to 70% |
| Time for maximum effectiveness | 4 weeks | B to 12 weeks |
| Remission of vaginal atrophy | Favorable effect | Favorable effect |
| Increase in bone mineral density | Favorable effect | Favorable effect |

| Side effects | | |
|---|---|---|
| Incidence of Irregular bleeding | 23% | 12% |
| Incidence of Spotting or licking | 44% | 22% |
| Incidence of endometrial hyperplasia with single estrogen | 27.3% | 3.2% |

### (Velasco-Murillo V. 2006. Op. Cit.)

There are several options to treat and cure this problem, for instance, US patent 3 733 407 discloses a method for a hormonal replacement method to relieve or prevent the symptoms of menopause, with the disadvantage that the daily dose causes the patient to leave treatment.

US patent 4 425 339 discloses a treatment method to relieve menopause symptoms administering estrogen and progestogen in a four-phase sequence, for a period of 100 days, with the disadvantage of favoring treatment abandonment.

US patent 4 900 734 discloses pharmaceutical compounds containing estradiol and progesterone for their oral administration, estradiol is contained in a dosing solution containing a micronized progesterone suspension administered in one capsule.

US patent 4 945 103 discloses a treatment method for women with premenstrual syndrome, through the administration of melatonin and progestogen, with the disadvantage that it has to be administered on a daily basis which favors treatment abandonment.

US patent 5 514 382 discloses a mineral and vitamin supplement of daily intake containing vitamin α-, p-carotene, niacin, ribloflavin, pantothenic acid, pyridoxine, biotin, aminobenzoic acid, inositol, iodine, iron, magnesium, manganese, molybdenum, selenium, zinc and bioflavonoids to relieve the symptoms of menopause, it has to be administered everyday with the disadvantage of favoring treatment abandonment.

The object of this invention is to provide an effective pharmaceutical compound or formulation, for the therapeutic use of hormonal therapy in women with uterus to mitigate the symptomatology and discomfort associated with the climacteric syndrome.

Another object of this invention is to provide an effective pharmaceutical formulation, for the hormonal therapeutic use in women with uterus, during the treatment of the symptomatology and to mitigate discomfort associated with clicmateric syndrome.

Another object of this invention is to provide an effective pharmaceutical formulation, for hormonal therapy in women with uterus, for climacteric syndrome, with a dosage form that allows the compliance of the treatment, for instance, monthly in the case of transdermal application.

Another object of this invention is to provide an effective pharmaceutical formulation for hormonal therapy in women with uterus, in the treatment of the symptomatology and discomfort associated with climacteric syndrome, in a low dose in order to minimize the risk of appearance of side effects, such as breast cancer.

Another object of the invention is to provide an effective pharmaceutical formulation for hormonal therapy in women with uterus to treat the symptomatology and discomfort associated with climacteric syndrome, in ultra-low doses in order to minimize the risk of undesirable effects such as breast cancer.

An additional object of this invention is to provide a pharmaceutical compound or formulation that can be applied parenterally, that is to say, in an intramuscular or subcutaneous manner.

### Brief description of the Drawings

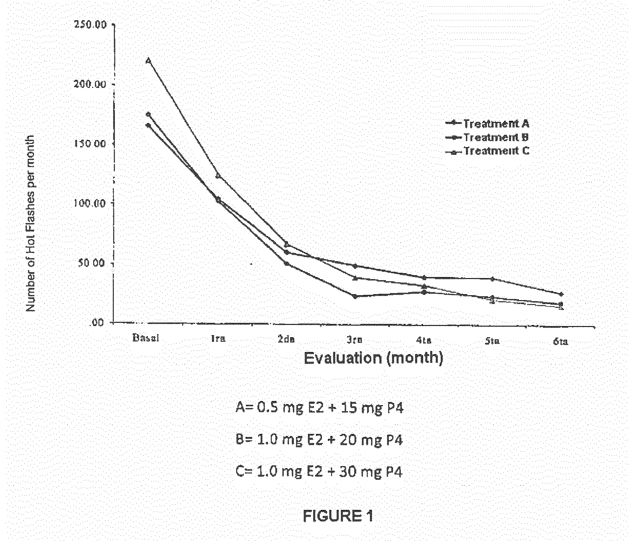

Figure 1 is a graphic of an evaluation of hot flashes per month in a period of six months.

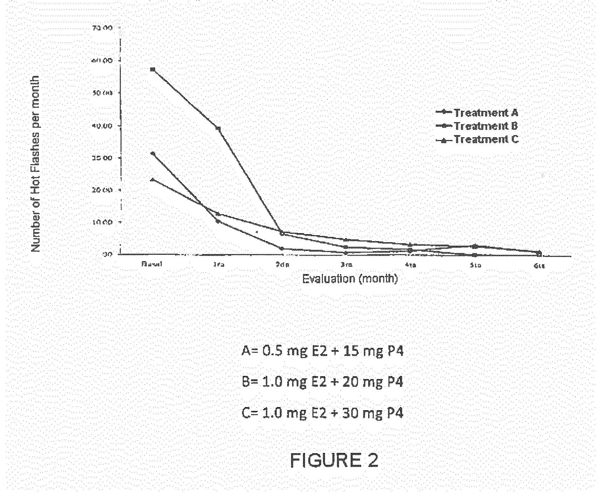

Figure 2 is a relative graphic of the evolution of hot flashes during the 6 months of the analysis (severe hot flashes).

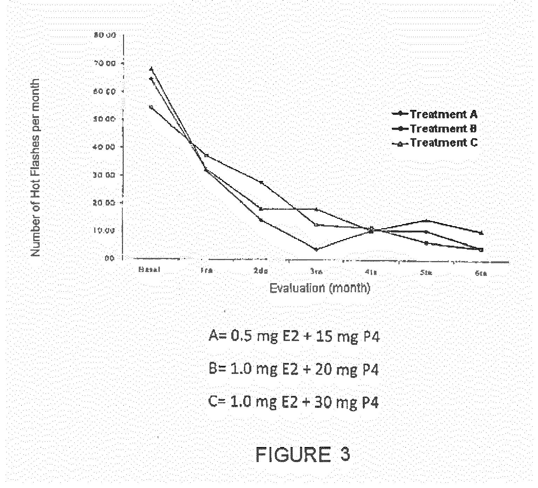

Figure 3 is a graphic related to the evolution of hot flashes during the 6 months of the analysis (moderate hot flashes).

### DETAILED DESCRIPTION OF THE INVENTION

The present invention consists of a parenteral pharmaceutical compound or formulation that can be administered intramuscularly in a suspension, of sustained release, of suspended estradiol particles and progesterone for hormonal replacement in female mammals, in low and ultra-low doses. The formulation contains estradiol particles at an interval of 5 to 100 micrometers, progesterone particles at 5 to 100 micrometers, a surfactant agent, an isosmotic agent, a viscosity increasing agent and one or more preservatives.

Any surfactant agent capable of modifying the surface tension is selected in order to moisturize the hydrophobic particles of estradiol and progesterone.

The isosmotic agent is chosen only if it has the capacity of providing the same isotonicity as cells.

A viscosity increasing agent is chosen only if it is capable of modifying the viscosity of the vehicle for the particles to be able to suspend and re-suspend.

A preservative is chosen only if is capable of inhibit microbial growth.

The agent to adjust pH is chosen only if it equilibrates the pH of the compound in physiological conditions.

The pharmaceutical compound can be injected and it is released in a sustained manner for a month in virtue of which the dose might be monthly.

The compound is achieved through the use of micro-particles such as those prepared in accordance with US patent 2005/0025827, however, in this case this is a monthly therapy, through an injectable compound in which the size of the esteridol and stable progesterone is from 5 to 100 micrometers which is attained through.

Below, in an expository manner, but not limited to, we give a series of examples focused in preparing the product, the form of obtaining of the estradiol and progesterone particles is provided for the formulation as well as the obtaining of the injectable.

### Examples to prepare estradiol and progesterone particles.

### Obtaining of estradiol particles.

Estradiol is melted and atomized by centrifugation in an atomizer by freezing at a temperature between 165 to 190°C. In the same equipment, formed micro drops are frozen at a temperature between -20 to -5°C.

The obtained solid particles are crystallized in accordance with the proceeding described in patent application US 2005/0025827, achieving stable forms from this active principle.

Afterwards, the obtained solid particles are classified in accordance with their sixe through an ultrasonic sieve, considering as adequate, those that have size between 1 and 100 micrometers.

The particles obtained from the previous step, are sterilized with ethylene oxide.

### Obtaining of progesterone particles.

Progesterone is melted and atomized by centrifugation, in an atomizer by freezing, at a temperature between 130 and 170°C. In the same equipment, the formed micro drops are frozen at a temperature between 60 and -20°C.

The obtained solid particles are classified in accordance with their sixe through an ultrasonic sieve, considering as adequate, those that have size between 1 and 100 micrometers.

Afterwards, the particles of interest are crystallized in an oven, at a temperature between 50 and 105°C, achieving stable forms of this active principle.

The particles obtained from the previous step, are sterilized with ethylene oxide.

In order to achieve the final product, these stable estradiol and progesterone particles are mixed with surfactant agent, an isosmotic agent and a preservative, in a preferred modality, as illustrated in the following examples.

Used components might be:

| | |
|---|---|
| Surfactant Agent | Polysorbate 20, polysorbate 80, Dioctyl sodium sulfosuccinate, polyoxyethylene resin oil. |
| Isosmotic Agent | Sodium chloride, lactose, trehalose, mannitol, glycerin, sucrose |
| Viscosity Increasing agent | Sodium Carboxymethyl Cellulose, polyethylene glycol 300, polyethylene glycol 400, polyethylene glycol 3350 |
| Preservative | Methylparaben, Propylparaben, Phenol, Thiomersal, m-Cresol, chlorobutanol, benzalkonium chloride, Benzyl alcohol, 2-Phenoxyethanol. |
| pH adjusting agent | Hydrochloric Acid, Phosphoric Acid, Sodium Hydroxide, Sulfuric Acid |

### Formulation Examples Including Estradiol and Progesterone

### Example 1.

### Syringe with powder:

0.25 mg of sterile estradiol particles and 1.50 mg of sterile progesterone particles obtained in accordance with the previous proceeding, mixed aseptically in a V mixer, upon obtaining of a homogeneous mixture. The obtained product is aseptically deposited in syringes, using a filling - stopper machine for syringes.

### Aqueous Vehicle

Is made from the following components:

| Component | Unitary Formula |
|---|---|
| Methylparaben | 0.69 mg |
| Propylparaben, | 0.08 mg |
| Mannitol | 24.00 mg |
| Sodium Carboxymethyl Cellulose | 0.38 mg |
| Polysorbate 80 | 0.10 mg |
| Water for injectables | 0.50 ml |
| Hydrochloric Acid 1.0 N | To adjust pH, if needed |

All of the raw materials are of pharmaceutical degree.

Parabens are dissolved in water for injectables, hot and in the same solution Mannitol and Sodium Carboxymethyl Cellulose are dissolved.

Separately, polysorbate is dissolved in water for injectables and both solutions are incorporated.

The pH of the solution is adjusted to a value between 5.0 and 6.5. This solution is sterilized by filtration and is filled aseptically with a vial in a machine for this purpose.

The product, a dispersion powder, is composed of the syringe with the powder and the vial with the vehicle. These components are incorporated into a sole product at the moment of its administration.

The obtaining of a stable form is achieved, which means an injectable compound, intramuscular or subcutaneous, of sole dose that maintains its physical, physical-chemical and microbiological characteristics for at least two years, in such a manner that therapeutic effects are not affected.

### Example 2.

Syringe with powder: 0.50mg of sterile estradiol particles and 15.0mg of sterile progesterone particles obtained in accordance with the previous proceeding, they are mixed aseptically in a V mixer, upon obtaining of a homogeneous mixture. The obtained product is aseptically deposited in syringes, using a filling - stopper machine for syringes.

### Aqueous vehicle.

The following components are used:

| Component | Unitary Formula |
|---|---|
| Methylparaben | 0.69 mg |
| Propylparaben | 0.08 mg |
| Mannitol | 24.00 mg |
| Sodium Carboxymethyl Cellulose | 0.38 mg |
| Polysorbate 80 | 0.10 mg |
| Water for injectables | 0.50 ml |
| Hydrochloric Acid 1.0 N | To adjust pH, if needed |

All of the raw materials are of pharmaceutical degree.

Parabens are dissolved in water for injectables, hot and in the same solution Mannitol and Sodium Carboxymethyl Cellulose are dissolved.

Separately, polysorbate is dissolved in water for injectables and both solutions are incorporated.

The pH of the solution is adjusted to a value between 5.0 and 6.5. This solution is sterilized by filtration and is filled aseptically with a vial in a machine for this purpose.

The product, a dispersion powder, is composed of the syringe with the powder and the vial with the vehicle. These components are incorporated into a sole product at the moment of its administration.
The obtaining of a stable form is achieved, which means an injectable compound, intramuscular or subcutaneous, of sole dose that maintains its physical, physical-chemical and microbiological characteristics for at least two years, in such a manner that therapeutic effects are not affected.

### Example 3.

### Syringe with powder:

1.00mg of sterile estradiol particles and 20.0mg of sterile progesterone particles obtained in accordance with the previous proceeding, they are mixed aseptically in a V mixer, upon obtaining of a homogeneous mixture. The obtained product is aseptically deposited in syringes, using a filling - stopper machine for syringes.

### Aqueous vehicle.

The following components are used:

| Component | Unitary Formula |
|---|---|
| Methylparaben | 1.37 mg |
| Propylparaben | 0.15 mg |
| Mannitol | 48.00 mg |
| Sodium Carboxymethyl Cellulose | 0.75 mg |
| Polysorbate 80 | 0.20 mg |
| Water for injectables | 1.0 ml |
| Hydrochloric Acid 1.0 N | To adjust pH, if needed |

All of the raw materials are of pharmaceutical degree.

Parabens are dissolved in water for injectables, hot and in the same solution Mannitol and Sodium Carboxymethyl Cellulose are dissolved.

Separately, polysorbate is dissolved in water for injectables and both solutions are incorporated.

The pH of the solution is adjusted to a value between 5.0 and 6.5. This solution is sterilized by filtration and is filled aseptically with a vial in a machine for this purpose.

The product, a dispersion powder, is composed of the syringe with the powder and the vial with the vehicle. These components are incorporated into a sole product at the moment of its administration.
The obtaining of a stable form is achieved, which means an injectable compound, intramuscular or subcutaneous, of sole dose that maintains its physical, physical-chemical and microbiological characteristics for at least two years, in such a manner that therapeutic effects are not affected.

### Example 4.

### Syringe with powder:

0.25mg of sterile estradiol particles and 15.0mg of sterile progesterone particles obtained in accordance with the previous proceeding, they are mixed aseptically in a V mixer, upon obtaining of a homogeneous mixture. The obtained product is aseptically deposited in syringes, using a filling - stopper machine for syringes.

### Aqueous vehicle.

The following components are used:

| Component | Unitary Formula |
|---|---|
| Polyethylene glycol 400 | 200 mg |
| polysorbate 80 | 2.41 mg |
| Mannitol | 16.0 mg |
| Water for injectables | 1.0 ml |
| Hydrochloric Acid 1.0 N | To adjust pH, if needed |

All of the raw materials are of pharmaceutical degree.

Polyethylene Glycol, mannitol and polysorbate are dissolved in water for injectables.

The pH of the solution is adjusted to a value between 5.0 and 6.5. This solution is sterilized by filtration and is filled aseptically with a vial in a machine for this purpose.

The product, a dispersion powder, is composed of the syringe with the powder and the vial with the vehicle. These components are incorporated into a sole product at the moment of its administration.

The obtaining of a stable form is achieved, which means an injectable compound, intramuscular or subcutaneous, of sole dose that maintains its physical, physical-chemical and microbiological characteristics for at least two years, in such a manner that therapeutic effects are not affected.

### Example 5.

### Syringe with powder:

1.00mg of sterile estradiol particles and 20.0mg of sterile progesterone particles obtained in accordance with the previous proceeding, they are mixed aseptically in a V mixer, upon obtaining of a homogeneous mixture. The obtained product is aseptically deposited in syringes, using a filling - stopper machine for syringes.

### Aqueous vehicle.

The following components are used:

| Component | Unitary Formula |
|---|---|
| Polyethylene glycol 3350 | 28.9 mg |
| Polysorbate 80 | 2.41 mg |
| Mannitol | 8.68 mg |
| Water for injectables | 1.0 ml |
| Hydrochloric Acid 1.0 N | To adjust pH, if needed |

All of the raw materials are of pharmaceutical degree.

Polyethylene Glycol, mannitol and polysorbate are dissolved in water for injectables.

The pH of the solution is adjusted to a value between 5.0 and 6.5. This solution is sterilized by filtration and is filled aseptically with a vial in a machine for this purpose.

The product, a dispersion powder, is composed of the syringe with the powder and the vial with the vehicle. These components are incorporated into a sole product at the moment of its administration.
The obtaining of a stable form is achieved, which means an injectable compound, intramuscular or subcutaneous, of sole dose that maintains its physical, physical-chemical and microbiological characteristics for at least two years, in such a manner that therapeutic effects are not affected.

### CLINICAL STUDIES

### Effectiveness

An Effectiveness and security clinical study was carried out in 103 peri and post-menopausal women with vasomotor and vulvovaginal symptomatology, administering three different suspension doses of Estradiol: Cholesterol/Progesterone Microspheres, administered every 30 ± 3 days, for 6 consecutive months:
0.5 mg of Estradiol/ 15 mg of Progesterone
1.0 mg of Estradiol/ 20 mg of Progesterone
1.0 mg of Estradiol/ 30 mg of Progesterone

Effectiveness and security results are presented below.

### Effect on vasomotor symptoms.

The Effectiveness of the three dose levels of Estradiol (E2)/Progesterone (P4): 0.5mg of E2/15 mg of P4, 1 mg of E2/20 mg of P4, 1 mg of E2/30 mg of P4, administered every 30 ± 3 days, in the relieving of vasomotor symptoms in frequency and intensity (moderate and severe) in peri and post-menopausal women between 40 and 65 years old, was evaluated in a 6 month study (n= 103). The three evaluated dose levels were not presented among them, statistically significant differences in the decrease in the. number of hot flashes at the third and sixth administration *, ** (p>0.05).

**Table 2. Number, average and percentage in the decrease of total hot flashes per treatment and administered dose.**

| Type of Treatment | Analysis | Evaluations (monthly) | | | | | | | p Value |
|---|---|---|---|---|---|---|---|---|---|
| | | Basal | 1 | 2 | 3* | 4 | 5 | 6** | |
| A 0.5 mg E₂/ 15mg P₄ | n Average (DE) | 38 165.84 (112.95) | 38 104.68 (72.91) | 38 60.34 (55.78) | 37 49.11 (47.89) | 35 39.97 (54.16) | 34 39.29 (48.22) | 34 27.09 (41.27) | p<0.05 |
| | Decrease Percentage | 0% | 37% | 64% | 70% | 76% | 76% | 84% | - |
| B 1.0 mg E₂/ 20mg P₄ | n Average (DE) | 29 174.76 (221.31) | 27 102.93 (114.72) | 26 50.73 (51.77) | 25 23.36 (23.98) | 24 27.46 (50.20) | 24 23.42 (58.72) | 24 18.54 (40.32) | p<0.05 |
| | Decrease Percentage | 0% | 41% | 71% | 87% | 84% | 87% | 89% | - |
| C 1.0 mg E₂/ 30mg P₄ | n Average (DE) | 36 220.19 (222.52) | 33 124.33 (105.03) | 32 67.28 (70.13) | 29 39.21 (42.17) | 26 32.88 (49.17) | 26 21.00 (34.24) | 26 16.23 (24.04) | p<0.05 |
| | Decrease Percentage | 0% | 44% | 69% | 82% | 85% | 90% | 93% | - |
| Friedman Test (p≤0.05) | | | | | | | | | |
| *p=0.056 upon comparison of the three groups in dose 3. | | | | | | | | | |
| **p=0.478 upon comparison of the three groups in dose 6. | | | | | | | | | |

The intensity of hot flashes evaluated at month 6 presented a statistically significant decrease (p<0.05) at three dose levels. In the evaluation at 3 months, the decrease of moderate hot flashes was greater in treatment B (1.0 mg of E2/20 mg of P4) when compared with treatments A and C (0.5mg of E2/15mg of P4 and 1.0mg of E2/ 30mg of P4 respectively) (p≤0.05).

At the end of the proceeding, there was no significant difference between the three treatments with regard to decrease proportions.

**Table 3. Change in the intensity of hot flashes in basal, intermediate and final evaluations in each type of treatment.**

| Severity of Hot Flashes | Treatments | | | | | | | | | | | |
|---|---|---|---|---|---|---|---|---|---|---|---|---|
| | A | | | | B | | | | C | | | |
| | Basal n=38 | Int n=37 | Final n=34 | p Value | Basal n=29 | Int n=25 | Final n=24 | p Value | Basal n=36 | Int n=29 | Final n=26 | p Value |
| Slight Average (DE) | 73.0 (65.3) | 25.9 (29.0) | 15.8 (27.0) | p<0.05 | 78.8 (131.9) | 18.7 (20.7) | 13.7 (33.3) | p<0.05 | 108.6 (118.2) | 24.2 (29.7) | 12.1 (22.2) | p<0.05 |
| Moderate Average (DE) | 69.5 (110.9) | 18.5 (24.1) | 10.2 (26.7) | p<0.05 | 64.6 (72.8) | 3.9 (6.8) | 4.0 (9.2) | p<0.05 | 54.4 (78. 4) | 12.6 (22.8) | 4.0 (9.0) | p<0.05 |
| Severe Average (DE) | 23.3 (50.3) | 4.7 (12.2) | 1.2 (4.1) | p<0.05 | 31.4 (62.8) | 0.7 (3.6) | 0.9 (3.7) | p<0.05 | 57.3 (107.1) | 2.4 (8.7) | 0.1 (0.4) | p<0.05 |
| Basal (-1 month), intermediate (3^{rd} month) and final (6^{th} month); Friedman Test (p≤0.05) | | | | | | | | | | | | |

### Effect on vulvovaginal symptoms

Vulvovaginal symptoms decreased considerably in the three groups (p<0.05).

Dysuria disappeared in the group of 1mg of Estradiol/ 30 of Progesterone (P4)m although it was not statistically relevant in the group of 1mg of Estradiol (E2)/ 20mg of Progesterone, probably because in this group, from the basal stage, the proportion was lower than groups A and C.

Dyspareunia had an important decrease in group B (1.0 mg of E2/20mg of P4) (p<0.05.

The proportion of volunteers with post-coital bleeding was low in relation to the rest of vulvovaginal symptoms in the basal stage and disappeared totally in the three treatment groups.

**Table 4. Modifications in vulvovaginal atrophy symptoms.**

| Symptoms and signs | Treatments | | | | | | | | | | | |
|---|---|---|---|---|---|---|---|---|---|---|---|---|
| | (A) 0.5mg E₂ and 15mg P₄ | | | | (B) 1.0mg E₂ and 20mg P₄ | | | | C 1.0mg E₂ and 30mg P₄ | | | |
| | Basal n=38 % | Int n=37 % | Final n=34 % | p Value | Basal n=29 % | Int n=25 % | Final n=24 % | p Value | Basal n=36 % | Int n=29 % | Final n=26 % | p Value |
| Vaginal Dryness | 50.0 | 24.3 | 17.6 | p<0.05 | 58.6 | 12.0 | 8.3 | p<0.05 | 36.1 | 31.0 | 23.1 | p>0.05 |
| Vulvar and vaginal irritation and pruritus | 42.1 | 24.3 | 14.7 | p<0.05 | 37.9 | 16.0 | 4.2 | p<0.05 | 30.6 | 13.8 | 15.4 | p<0.05 |
| Dysuria | 28.9 | 2.7 | 2.9 | p<0.05 | 10.3 | 4.0 | 0.0 | p<0.05 | 19.4 | 10.3 | 0.0 | p<0.05 |
| Dyspareunia | 18.4 | 5.4 | 2.9 | p>0.05 | 17.2 | 4.0 | 0.0 | p<0.05 | 8.3 | 0.0 | 3.8 | p>0.05 |
| Post-coital Bleeding | 2.6 | 2.7 | 0.0 | p>0.05 | 6.9 | 0.0 | 0.0 | p>0.05 . | 5.6 | 0.0 | 0.0 | p>0.05 |
| Basal: -1month, int: 3months, final: 6 months. Cochran's Test (p≤0.05). | | | | | | | | | | | | |

### Quality of Life

The evaluation of the quality of life was made through the Utian Scale. Results are presented in Table 5.

**Table 5. Evaluation of Quality of Life (Utian Scale)**

| Domain | Average Reference Value | Treatments | | | | | | | | | | | |
|---|---|---|---|---|---|---|---|---|---|---|---|---|---|
| | | (A) 0.5mg E₂ and 15mg P₄ | | | | (B) 1.0mg E₂ and 20mg P₄ | | | | (C) 1.0mg E₂ and 30mg P₄ | | | |
| | | Basal | Int | Final | p | Basal | Int | Final | p | Basal | Int | Final | p |
| Occupation a1 Mean (SD) | 25 | 26.1 (8.0) | 30.7 (5.5) | 29.7 (7.0) | 0.00 1 | 28.0 (8.0) | 29.5 (6.1) | 29.8 (5.6) | 0.48 1 | 22.6 (9.6) | 27.7 (6.0) | 28.5 (5.6) | 0.003 |
| Health Mean (SD) | 21 | 21.3 (5.4) | 22.9 (4.4) | 23.1 (4.0) | 0.67 1 | 22.0 (5.1) | 23.3 (4.8) | 22.7 (4.2) | 0.47 1 | 21.8 (6.1) | 22.0 (4.3) | 21.6 (3.1) | 0.674 |
| Emotional Mean (SD) | 20 | 18.2 (4.7) | 17.6 (3.7) | 17.0 (3.3) | 0.03 1 | 19.3 (4.3) | 18.6 (3.7) | 17.1 (4.1) | 0.004 | 20.2 (5.2) | 18.2 (4.2) | 18.2 (4.3) | 0.104 |
| Sexual Mean (SD) | 8 | 9.6 (3.5) | 10.7 (2.4) | 10.4 (2.3) | 0.43 4 | 9.4 (3.6) | 10.2 (3.1) | 10.5 (2.7) | 0.33 0 | 9.1 (2.9) | 10.5 (2.1) | 10.4 (2.6) | 0.198 |
| Total Mean (SD) | 74 | 75.0 (16.8) | 81.8 (10.3) | 80.0 (11.9) | 0.17 5 | 78.6 (12.2) | 81.6 (10.5) | 80.1 (11.1) | 0.86 7 | 73.7 (17.7) | 78.3 (9.9) | 78.6 (10.1) | 0.070 |
| Basal (-1 month), intermediate (3^{rd} month) and final (6^{th} month); Friedman Test (p≤0.05) | | | | | | | | | | | | | |

The Quality of Life (Utian Scale) of the participants showed a significant increase (p<0.05) in the occupational (treatments A and C) and emotional (treatments A and B) domains, health and sexual domains did not show a significant improvement (p>0.05).

### Security

In a monitoring study at 6 months carried out in 103 peri and post-menopausal women (with intact uterus) which were treated every 30 ± 3 days with an IM injection of an aqueous suspension of microspheres of Estradiol/Progesterone, endometrial security was evaluated using the following parameters: endometrial thickness identified through endovaginal ultrasound and endometrial biopsy.

### Result of ultrasound studies.

Basal endometrial thickness in the peri-menopausal group was 5.4mm and the final value 4.9mm. In the post-menopausal group, basal endometrial thickness was 3.4mm and the final value 3.0mm.

### Endometrial Biopsy Result

A total of 55 endometrial biopsies were carried out, in which, after 6 months of continuous treatment, no histological report of endometrial hyperplasia was found.

Table 6 presents the results of this evaluation.

**Table 6. Incidence of endometrial hyperplasia after 6 months of treatment. ITT Population.**

| Characteristics | (A) 0.5 mg E2/15 mg P4 n=38 | (B) 1.0mg E2/20 mg P4 n=29 | (C) 1.0mg E2/30 mg P4 n=36 |
|---|---|---|---|
| No. of volunteers with evaluable biopsies at 6 months | 22 | 15 | 18 |
| No. (%) of volunteers with hyperplasia at 6 months. | 0.0 | 0.0 | 0.0 |

### Effect on uterine bleeding or spotting pattern.

The effects of the injectable suspension of microspheres of Estradiol/Progesterone on the uterine bleeding or spotting, were registered for 6 consecutive months in the patient's journal and reported in the programmed visits. Results are shown in Tables 7 and 8.

In 58 peri-menopausal women, the duration of the spotting/bleeding days of their basal pattern, was maintain without changes throughout the six treatment cycles with the three doses.

**Table 7. Average spotting/bleeding days in each evaluation per type of treatment in peri-menopausal women.**

| Variables | Analysis | Evaluation (month) | | | | | | |
|---|---|---|---|---|---|---|---|---|
| | | Basal and beginning of treatment | 1^{st} | 2^{nd} | 3^{rd} | 4^{th} | 5^{th} | 6^{th} |
| Treatment A n=22 | | | | | | | | |
| Spotting | n | 9 | 11 | 11 | 10 | 10 | 9 | 8 |
| | average days | 3.0 | 3.2 | 5.1 | 3.3 | 2.5 | 2.7 | 2.1 |
| | (min-max) | (1.0-6.0) | (2.0-5.0) | (2.0-18.0) | (1.0-9.0) | (1.0-4.0) | (1.0-4.0) | (1.0-4.0) |
| Bleeding | n | 8 | 11 | 12 | 12 | 10 | 6 | 9 |
| | average days | 3.4 | 4.1 | 3.9 | 4.0 | 3.8 | 3.2 | 4.0 |
| | (min-max) | (1.0-5.0) | (1.0-8.0) | (1.0-10.0) | (2.0-7.0) | (3.0-7.0) | (1.0-5.0) | (3.0-7.0) |

| Treatment B | | | | | | | | |
|---|---|---|---|---|---|---|---|---|
| n=15 | | | | | | | | |
| Spotting | n | 8 | 11 | 11 | 7 | 11 | 9 | 9 |
| | average days | 3.6 | 4.4 | 3.4 | 4.3 | 3.1 | 3.1 | 2.6 |
| | (min-max) | (2.0-8.0) | (2.0-13.0) | (2.0-5.0) | (2.0-12.0) | (2.0-6.0) | (2.0-5.0) | (1.0-4.0) |
| Bleeding | n | 8 | 11 | 9 | 8 | 10 | 9 | 11 |
| | average days | 3.0 | 3.5 | 2.9 | 3.3 | 2.9 | 3.0 | 3.8 |
| | (min-max) | (1.0-5.0) | (1.0-7.0) | (1.0-5.0) | (2.0-4.0) | (1.0-4.0) | (1.0-7.0) | (1.0-9.0) |

| Treatment C n=21 | | | | | | | | |
|---|---|---|---|---|---|---|---|---|
| Spotting | n | 7 | 10 | 11 | 9 | 9 | 10 | 10 |
| | average days | 4.9 | 4.5 | 4.2 | 5.6 | 5.2 | 5.3 | 4.0 |
| | (min-max) | (3.0-6.0) | (1.0-14.0) | (1.0-9.0) | (1.0-14.0) | (1.0-9.0) | (1.0-21.0) | (1.0-11.0) |
| Bleeding | n | 7 | 8 | 8 | 7 | 10 | 7 | 8 |
| | average days | 3.4 | 3.8 | 3.9 | 3.9 | 3.7 | 2.6 | 2.9 |
| | (min-max) | (1.0-5.0) | (2.0-7.0) | (3.0-7.0) | (2.0-7.0) | (1.0-6.0) | (1.0-4.0) | (1.0-7.0) |

Of the 45 post-menopausal women, the group which presented the biggest proportion of spotting/bleeding episodes were those that received the treatment of 1 mg of Estradiol/30mg of Progesterone (6 out of every 15 women) with duration of 15 days.

**Table 8. Average spotting/bleeding days in each evaluation per type of treatment in post-menopausal women.**

| Variables | Analysis | Evaluation (month) | | | | | | |
|---|---|---|---|---|---|---|---|---|
| | | Basal and beginning of treatment | 1^{st} | 2^{nd} | 3^{rd} | 4^{th} | 5^{th} | 6^{th} |
| Treatment A n=16 | | | | | | | | |
| Spotting | n | 1 | 1 | - | 1 | - | - | 1 |
| | average days | 2.0 | 1.0 | - | 2.0 | - | - | 3.0 |
| | (min-max) | (2.0) | (1.0) | - | (2.0) | - | - | (3.0) |
| Bleeding | n | - | - | - | 1 | - | - | 1 |
| | average days | - | - | - | 4.0 | - | - | 1.0 |
| | (min-max) | - | - | - | (4.0) | - | - | (1.0) |

| Treatment B n=14 | | | | | | | | |
|---|---|---|---|---|---|---|---|---|
| Spotting | n | 1 | - | 1 | 3 | 2 | 3 | 1 |
| | average days | 5.0 | - | 3.0 | 2.3 | 3.5 | 2.3 | 5.0 |
| | (min-max) | (5.01 | - | (3.0) | (1.0-5.0) | (1.0-6.0) | (1.0-4.0) | (5.0) |
| Bleeding | n | - | - | 1 | 1 | - | 1 | - |
| | average days | - | - | 4.0 | 1.0 | - | 2.0 | - |
| | (min-max) | - | - | (4.0) | (1.0) | - | (2.0) | - |

| Treatment C n=15 | | | | | | | | |
|---|---|---|---|---|---|---|---|---|
| Spotting | n | - | 2 | 3 | 6 | 4 | 5 | 6 |
| | average days | - | 2.5 | 2.0 | 4.8 | 3.0 | 3.6 | 3.2 |
| | (min-max) | - | (2.0-3.0) | (1.0-3.0) | (3.0-6.0) | (2.0-4.0) | (2.0-5.01 | (1.0-9.0) |
| Bleeding | n | - | - | 2 | 2 | 2 | 2 | 6 |
| | average days | - | - | 3.5 | 1.0 | 3.0 | 3.0 | 2.2 |
| | (min-max) | - | - | (2.0-5.0) | (1.0) | (2.0-4.0) | (3.0) | (1.0-4.0) |

### Local and Systemic Security

The three treatments showed successful local and systemic tolerability after the administration of 545 monthly injections. Adverse events observed with very common frequency (> 10%) and common (> 10%, <10%) are presented in Table 9.

Local tolerability, the pain in the injection site was the event classified as very common and inflammation in the injection site was observed as a common event in the three evaluated treatments.

In relation to the adverse systemic events, headache followed by mastalgia were the events observed as very common; irritability, sickness, anxiety and blurry vision were observed as common events throughout the study.

**Table 9 Side effects with very common and common classification**

| Affected System | Events | Very Common (≥ 10%) | | | Common (≥ 1% to <10%) | | |
|---|---|---|---|---|---|---|---|
| | | A n=38 | B n=29 | C n=36 | A n=38 | B n=29 | C n=36 |
| General | Cramps | **--** | -- | -- | ✔ | -- | -- |
| | Headache | ✔ | ✔ | ✔ | -- | **--** | **--** |
| Cardiovascular | Palpitations | -- | -- | **--** | -- | ✔ | -- |
| | Peripheral vascular insufficiency | -- | -- | **--** | -- | -- | ✔ |
| | Tachycardia | -- | -- | -- | -- | ✔ | -- |
| Digestive | Hypogastric Colic | -- | -- | **--** | ✔ | -- | -- |
| | Anorexia | -- | -- | -- | -- | ✔ | -- |
| | Increased appetite | -- | -- | -- | -- | ✔ | -- |
| | Nausea | -- | -- | -- | -- | -- | ✔ |
| Nutritional Metabolic | Dyslipidemia | -- | -- | -- | ✔ | -- | ✔ |
| | Hyperlipidemia | -- | -- | -- | -- | -- | ✔ |
| Skeletal-Muscle | Arthralgia | -- | -- | -- | ✔ | -- | ✔ |
| | Myalgia | -- | -- | -- | ✔ | -- | ✔ |
| | Bone pain | -- | -- | -- | -- | -- | ✔ |
| Nervous | Depression | -- | -- | -- | ✔ | -- | -- |
| | Aggressiveness | -- | -- | -- | -- | -- | ✔ |
| | Anguish | -- | -- | -- | -- | -- | ✔ |
| | Anxiety | -- | -- | -- | -- | -- | ✔ |
| | Nocturnal Phobia | -- | -- | -- | -- | -- | ✔ |
| | Insomnia | -- | -- | -- | ✔ | -- | ✔ |
| | Irritability | -- | -- | -- | ✔ | ✔ | ✔ |
| | Sickness | -- | -- | -- | ✔ | ✔ | ✔ |
| | Nervousness | -- | -- | -- | ✔ | ✔ | ✔ |
| Dermatological (in the site of application) | Burning | -- | -- | -- | -- | ✔ | ✔ |
| | Increase | -- | -- | -- | ✔ | ✔ | ✔ |
| | Pain | ✔ | ✔ | ✔ | -- | -- | -- |
| | Ecchymosis | -- | -- | -- | -- | -- | ✔ |
| | Induration | -- | -- | -- | -- | -- | ✔ |
| | Edema | -- | -- | -- | ✔ | -- | -- |
| | Erythema | -- | -- | -- | -- | ✔ | -- |
| Sense Organs | Blurry Vision | -- | -- | -- | ✔ | ✔ | ✔ |
| | Visual Alterations | -- | -- | -- | -- | -- | ✔ |
| Genitourinary | Increase of endometrial thickness | -- | -- | -- | ✔ | ✔ | -- |
| | Increase of menstrual bleeding | -- | -- | -- | -- | -- | ✔ |
| | Menstrual Colic | -- | -- | -- | -- | ✔ | -- |
| | Fibrocystic changes of breasts | -- | -- | -- | ✔ | -- | -- |
| | Cystic decay | -- | -- | -- | -- | ✔ | -- |
| | Pelvic Inflammation | -- | ✔ | -- | ✔ | -- | ✔ |
| | Mastalgia | ✔ | -- | ✔ | -- | ✔ | -- |
| | Water retention | -- | **--** | -- | ✔ | ✔ | -- |

### EFFECTIVENESS AND SECURITY SUMMARY

### Effectiveness

Results show that the three treatments were effective to reduce climacteric symptoms in an approximate period of 1 month, said reduction was maintained with a descending tendency until the end of the study.

The reduction of vasomotor symptoms at the end of the sixth moth of treatment was:
84% with the dose of 0.5 mg of E2/15 mg of P4
89% with the dose of 1mg of E2/20 mg of P4
93% with the dose of 1mg of E2/30 mg of P4

The treatment with 1 mg of E2/20 mg of P4 maintained a better Effectiveness and security equilibrium in comparison with the other two treatments.

Vulvovaginal symptoms showed tendency to decrease with the 3 treatments, with significant statistical difference in the treatment with 1mg of E2/20mg of P4.

The Quality of Life (measured in UTLAN scale) of women participating in the study has a significant improvement (p<0.05) in the occupational (treatments A and C) and emotional (treatments A and B) domains and in the domain of health and sex there was no significant improvement (p>0.05).

### Security

It was evaluated through the recording of spotting/bleeding patterns and the adverse effects reflected in tolerability; local, systemic and to change of endometrial conditions.

Peri-menopausal women preserved a normal menstrual pattern with the three treatments. The frequency of the spotting/bleeding episodes decreased in the group of post-menopausal women that received the treatment with a dose of 0.5mg of E2/15mg of P4 and 1.0mg of E2/20 mg of P4 in comparison with the dose of 1.0 mg of E2/30 mg of P4.

The 3 treatments has acceptable local and systemic tolerability evaluated in a total of 545 intramuscular administrations.

In the tolerability evaluation, the most common symptom was pain in the site of application, in the treatment of 1.0mg of E2/30 mg of P4 in 6.29%, treatment 1.0mg of E2+20mg of P4 in 5.26% and in treatment 0.5mg of E2/15mg of P4 in 4.1%, but in no case was it a reason for abandonment.

Systemic adverse events with definite relation , 8 out of 317 total, represented 2.5 % of the total of the recorded events. In the first place of frequency were registered: Myalgia (3 of 317= 0.95%) in reference to the abdomen/pelvic group and in second place mastalgia (2 of 317=0.63%), as the most frequent events with relation to Estradiol and Progesterone.

The events that caused abandonment of the study were headache/visual disorders (4/103 volunteers) and one case of probable exacerbation of venous insufficiency.

No compromise of endometrial health was observed with the use of treatments in the analyzed sample. Endometrial security evaluation was carried out through ultrasound in a sub-sample of volunteers through endometrial biopsy. The result of the ultrasound showed that the average endometrial thickness was inferior to 5mm both in peri-menopausal and post-menopausal women and endometrial biopsies reported no appearing of hyperplasia.

No hyperplasia observed after six months of continuous administration of the three treatments.

## Claims

1. A parenteral pharmaceutical formulation in suspension, of sustained release, containing suspended estradiol and progesterone particles suitable for hormonal replacement in female mammals in low or ultra-low dose, in the form of an injectable suspension formed by estradiol particles, progesterone particles, a surfactant agent, an isosmotic agent, a viscous increasing agent and one or more preservatives, **characterised in that**:
the low dose formulation comprises between 0.5mg of estradiol and 75mg of progesterone to 1.0 mg of estradiol and 20 mg of progesterone, and
the ultra-low dose formulation comprises 0.25 to 0.50mg of estradiol and 15.0mg of progesterone.

2. A parenteral pharmaceutical formulation in accordance with claim 1, in which estradiol particles are of a size between 1 and 100 micrometers.

3. A parenteral pharmaceutical formulation in accordance with claim 1, in which progesterone particles have a size between 1 and 100 micrometers.

4. A parenteral pharmaceutical formulation in accordance with claim 1, in which the obtained product is a suspension.

5. A parenteral pharmaceutical formulation in accordance with claims 1 to 4, in which the product is applied in a parenteral pharmaceutical form.

6. A parenteral pharmaceutical formulation in accordance with claim 5, in which the parenteral pharmaceutical form is intramuscular.

7. A parenteral pharmaceutical formulation in accordance with claim 5, in which the parenteral pharmaceutical form is subcutaneous.

8. A parenteral pharmaceutical formulation in accordance with claim 5, in which the parenteral pharmaceutical form is intradermal.

9. A parenteral pharmaceutical formulation in accordance with claim 1, in which the surfactant agents are selected from the group that consists of: polysorbate 20 polysorbate 80, dioctyl sodium sulfosuccinate and polyoxyethylene resin oil.

10. A parenteral pharmaceutical formulation in accordance with claim 1, in which the isosmotic agents are selected from the group that consists of: sodium chloride, lactose, trehalose, mannitol, glycerin and sucrose.

11. A parenteral pharmaceutical formulation in accordance with claim 1, in which preservatives are chosen from the group that consists of: Methylparaben, Propylparaben, Phenol, Thiomersal, m-Cresol, chlorobutanol, benzalkonium chloride, Benzyl alcohol and 2- Phenoxyethanol.

12. A parenteral pharmaceutical formulation in accordance with claim 1, in which the viscosity increasing are chosen from the group that consists of: Sodium Carboxymethyl Cellulose, polyethylene glycol 300, polyethylene glycol 400, polyethylene glycol 3350.

13. A parenteral pharmaceutical formulation in accordance with claim 12, in which the pH of the injectable form is between 4 and 7.

## Patentansprüche

1. Parenterale pharmazeutische Formulierung in Suspension mit kontinuierlicher Freisetzung, die suspendierte Östradiol- und Progesteronpartikel enthält, die als Hormonersatz bei weiblichen Säugetieren in niedriger oder ultraniedriger Dosis geeignet ist, in Form einer injizierbaren Suspension, die durch Östradiolpartikel, Progesteronpartikel, ein Tensid, ein isosmotisches Mittel, ein viskositätsteigerndes Mittel und ein oder mehrere Konservierungsmittel gebildet ist, **dadurch gekennzeichnet, dass**:
die niedrigdosierte Formulierung zwischen 0,5 mg Östradiol und 75 mg Progesteron und 1,0 mg Östradiol und 20 mg Progesteron umfasst, und
die ultraniedrigdosierte Formulierung 0,25 bis 0,50 mg Östradiol und 15,0 mg Progesteron umfasst.

2. Parenterale pharmazeutische Formulierung nach Anspruch 1, wobei Östradiolpartikel eine Größe zwischen 1 und 100 Mikrometern aufweisen.

3. Parenterale pharmazeutische Formulierung nach Anspruch 1, wobei Progesteronpartikel eine Größe zwischen 1 und 100 Mikrometern aufweisen.

4. Parenterale pharmazeutische Formulierung nach Anspruch 1, wobei das erhaltene Produkt eine Suspension ist.

5. Parenterale pharmazeutische Formulierung nach Anspruch 1 bis 4, wobei das Produkt in parenteraler pharmazeutischer Form angewandt wird.

6. Parenterale pharmazeutische Formulierung nach Anspruch 5, wobei die parenterale pharmazeutische Form intramuskulär ist.

7. Parenterale pharmazeutische Formulierung nach Anspruch 5, wobei die parenterale pharmazeutische Form subkutan ist.

8. Parenterale pharmazeutische Formulierung nach Anspruch 5, wobei die parenterale pharmazeutische Form intradermal ist.

9. Parenterale pharmazeutische Formulierung nach Anspruch 1, wobei die Tenside aus der Gruppe ausgewählt sind, die besteht aus: Polysorbat 20, Polysorbat 80, Dioctylnatriumsulfosuccinat und Polyoxyethylenrizinusöl.

10. Parenterale pharmazeutische Formulierung nach Anspruch 1, wobei die isosmotischen Mittel aus der Gruppe ausgewählt sind, die besteht aus: Natriumchlorid, Laktose, Trehalose, Mannitol, Glycerin und Saccharose.

11. Parenterale pharmazeutische Formulierung nach Anspruch 1, wobei Konservierungsmittel aus der Gruppe ausgewählt sind, die besteht aus: Methylparaben, Propylparaben, Phenol, Thiomersal, m-Cresol, Chlorbutanol, Benzalkoniumchlorid, Benzylalkohol und 2-Phenoxyethanol.

12. Parenterale pharmazeutische Formulierung nach Anspruch 1, wobei die viskositätsteigernden Mittel aus der Gruppe ausgewählt sind, die besteht aus: Natriumcarboxymethylcellulose, Polyethylenglykol 300, Polyethylenglykol 400, Polyethylenglykol 3350.

13. Parenterale pharmazeutische Formulierung nach Anspruch 12, wobei der pH-Wert der injizierbaren Form zwischen 4 und 7 liegt.

## Revendications

1. Formulation pharmaceutique parentérale en suspension, à libération entretenue, contenant des particules d'œstradiol et de progestérone en suspension, appropriées pour le remplacement hormonal chez des mammifères femelles à faible ou ultra-faible dose, sous la forme d'une suspension injectable formée par des particules d'œstradiol, des particules de progestérone, un agent tensio-actif, un agent isosmotique, un agent d'augmentation de la viscosité et un ou plusieurs conservateurs, **caractérisée par le fait que** :
la formulation à faible dose comprend entre 0,5 mg d'œstradiol et 75 mg de progestérone et 1,0 mg d'œstradiol et 20 mg de progestérone, et
la formulation à ultra-faible dose comprend 0,25 à 0,50 mg d'œstradiol et 15,0 mg de progestérone.

2. Formulation pharmaceutique parentérale selon la revendication 1, dans laquelle les particules d'œstradiol sont d'une dimension entre 1 et 100 micromètres.

3. Formulation pharmaceutique parentérale selon la revendication 1, dans laquelle les particules de progestérone ont une dimension entre 1 et 100 micromètres.

4. Formulation pharmaceutique parentérale selon la revendication 1, dans laquelle le produit obtenu est une suspension.

5. Formulation pharmaceutique parentérale selon l'une des revendications 1 à 4, dans laquelle le produit est appliqué sous une forme pharmaceutique parentérale.

6. Formulation pharmaceutique parentérale selon la revendication 5, dans laquelle la forme pharmaceutique parentérale est intramusculaire.

7. Formulation pharmaceutique parentérale selon la revendication 5, dans laquelle la forme pharmaceutique parentérale est sous-cutanée.

8. Formulation pharmaceutique parentérale selon la revendication 5, dans laquelle la forme pharmaceutique parentérale est intradermique.

9. Formulation pharmaceutique parentérale selon la revendication 1, dans laquelle les agents tensio-actifs sont choisis dans le groupe qui consiste en : polysorbate 20, polysorbate 80, dioctyl sulfosuccinate de sodium et huile de ricin polyoxyéthylénée.

10. Formulation pharmaceutique parentérale selon la revendication 1, dans laquelle les agents isosmotiques sont choisis dans le groupe qui consiste en : chlorure de sodium, lactose, tréhalose, mannitol, glycérol et saccharose.

11. Formulation pharmaceutique parentérale selon la revendication 1, dans laquelle les conservateurs sont choisis dans le groupe qui consiste en : Méthylparabène, Propylparabène, Phénol, Thiomersal, m-Crésol, chlorobutanol, chlorure de benzalkonium, Alcool benzylique et 2-Phénoxyéthanol.

12. Formulation pharmaceutique parentérale selon la revendication 1, dans laquelle les agents augmentant la viscosité sont choisis dans le groupe qui consiste en : Carboxyméthyl cellulose sodique, polyéthylène glycol 300, polyéthylène glycol 400, polyéthylène glycol 3350.

13. Formulation pharmaceutique parentérale selon la revendication 12, dans laquelle le pH de la forme injectable est entre 4 et 7.
